# EUROPÄISCHE PATENTSCHRIFT

(11) **EP 0 994 112 B1**
(45) Veröffentlichungstag und Bekanntmachung des Hinweises auf die Patenterteilung: **24.07.2002**
(21) Anmeldenummer: 99117240.4
(22) Anmeldetag: 02.09.1999
(51) Int. Cl.: C07D 307/33

(54) **Verfahren zur Abtrennung von Verunreinigungen aus 3-(2 -Acetoxy-ethyl)-dihydro-2(3H)furanon**
Method for separating contaminants from 3-(2'-acetoxy-ethyl)-dihydro-2(3h)furanone
Procédé pour la séparation d'impuretés dans la Préparation de la 3-(2-acetoxyethyl)-dihydro-2(3h)furanone

(30) Priorität: 10.09.1998 DE 19841520
(43) Veröffentlichungstag der Anmeldung: 19.04.2000
(73) Patentinhaber: BASF AKTIENGESELLSCHAFT, 67056 Ludwigshafen (DE)
(72) Erfinder: Maywald, Volker, Dr., 67071 Ludwigshafen (DE); Hartmann, Horst, 67459 Böhl-Iggelheim (DE); Götz, Norbert, Dr., 67547 Worms (DE); Reissenweber, Gernot, Dr., 67459 Böhl-Iggelheim (DE); König, Hartmann, Dr., 69115 Heidelberg (DE)
(74) Vertreter: Kinzebach, Werner, Dr.

(56) Entgegenhaltungen:
- EP-A- 0 584 631
- WO-A-99/29680
- US-A- 5 350 863

## Beschreibung

Die vorliegende Erfindung betrifft ein Verfahren zur Abtrennung unerwünschter Verunreinigungen aus 3-(2'-Acetoxyethyl)-dihydro-2(3H)furanon der Formel I

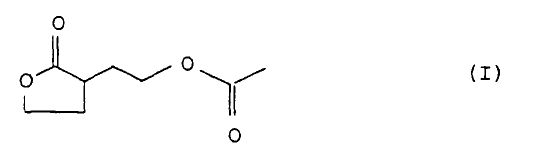

3-(2'-Acetoxyethyl)-dihydro-2(3H)furanon ist ein Ausgangsprodukt zur Herstellung von Tetrahydropyran-4-carbonsäure-methylester, welcher seinerseits ein Vorprodukt bei der Herstellung von Pflanzenschutzmitteln ist.

3-(2'-Acetoxyethyl)-dihydro-2(3H)furanon wird z.B. nach an sich bekannten und z.B. in der US-A 5,350,863 beschriebenen Verfahren ausgehend von Acetessigsäuremethylester und Ethylenoxid hergestellt. Eine andere Verfahrensvariante, nach der 3-(2'-Acetoxyethyl)-dihydro-2(3H)furanon erhältlich ist, ist in Dokl.Akad. NAuk SSSR 27, 956-959 (1940) und der US-A 5,283,326 beschrieben.

Nach beiden Varianten entsteht das gewünschte 3-(2'-Acetoxyethyl)-dihydro-2(3H)furanon im Gemisch mit einer Reihe unerwünschter Nebenkomponenten, insbesondere dem isomeren Dihydro-3-(2-methyl-1,3-dioxolan-2-yl)-2(3H)-furanon II

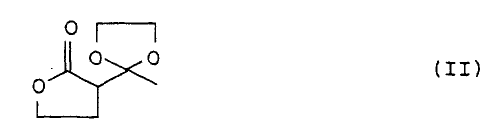

Wird das derart verunreinigte I in einer kontinuierlichen Gasphasenreaktion mit Methanol in Gegenwart saurer Katalysatoren zu Tetrahydropyran-4-carbonsäuremethylester umgesetzt, so werden deutlich niedrigere Ausbeuten und kürzere Katalysatorstandzeiten erhalten als bei Einsatz eines hochreinen Produkts ohne die Nebenkomponenten. Dies ist wahrscheinlich darauf zurückzuführen, daß die meisten Nebenkomponenten empfindliche Acetalgruppen enthalten, die sich an der Katalysatoroberfläche zu oligomeren und polymeren Produkten zersetzen.

Die Abtrennung der unerwünschten Nebenkomponenten, insbesondere des isomeren Dihydro-3-(2-methyl-1,3-dixolan-2-yl)-2(3H)-furanons II ist durch einfache diskontinuierliche Destillation bzw. Rektifikation nicht ausreichend zu realisieren. Darüber hinaus werden während der diskontinuierlichen Destillation bzw. Rektifikation des die unerwünschten Verunreinigungen enthaltenden 3-(2'-Acetoxyethyl)-dihydro-2(3H)furanons I weitere, schwer abtrennbare Leicht- und Zwischensieder gebildet, die das im Destillat anfallende gereinigte I wieder verunreinigen.

Es bestand daher die Aufgabe, ein Verfahren zur Verfügung zu stellen, nach welchem sowohl unerwünschte Nebenkomponenten, insbesondere II als auch neu gebildete Leicht- und Zwischensieder aus Mischungen mit I entfernt werden können.

Diese Aufgabe wurde erfindungsgemäß gelöst durch ein Verfahren zur Abtrennung von Verunreinigungen aus 3-(2'-Acetoxyethyl)-dihydro-2(3H)furanon (I), wobei das die unerwünschten Verunreinigungen enthaltende 3-(2'-Acetoxyethyl)-dihydro-2(3H)furanon zunächst in an sich bekannter Weise durch Acetylierung von 3-(2'-Hydroxyethyl)-dihydro-2(3H)furanon hergestellt wird und anschließend das so erhaltene Produkt einer Destillation oder Rektifikation unterworfen wird, wobei die Destillation oder Rektifikation mehrstufig derart durchgeführt wird, daß schwersiedende Verunreinigungen in einer ersten Stufe abgetrennt werden, anschließend das über Kopf abgezogene Produkt einer weiteren Stufe unterworfen wird, in der Leicht- und Zwischensieder über Kopf abgezogen und das gewünschte reine I als Sumpfprodukt erhalten wird.

Überraschenderweise wird mit dem erfindungsgemäßen Verfahren ein signifikant höherer Reinheitsgrad des gewünschten 3-(2'-Acetoxyethyl)-dihydro-2(3H)furanon (I) erhalten, als bei der üblichen Reihenfolge bei der Destillation bzw. Rektifikation, bei der Leicht- und Zwischensieder vor den Schwersiedern abgetrennt und das Wertprodukt am Kopf der Kolonne abgezogen wird. Ursache hier-für ist die Bildung von Leicht- und Zwischensiedern bei der Abtrennung der Schwersieder, die bei der üblichen Reihenfolge der Stufen nicht mehr abgetrennt werden können und die Reinheit des Kopfprodukts herabsetzen.

Bevorzugte Ausführungsformen des erfindungsgemäßen Verfahrens sind den Unteransprüchen und der nachfolgenden Beschreibung zu entnehmen.

Ausgangspunkt des erfindungsgemäßen Verfahrens ist das nach bekannten und in der Einleitung beschriebenen Verfahren erhältliche Gemisch aus 3-(2'-Acetoxyethyl)-dihydro-2(3H)furanon (nachfolgend mit I bezeichnet) und unerwünschten Verunreinigungen.

Dieses Gemisch wird im allgemeinen durch Acetylierung von 3-(2'-Hydroxyethyl)-dihydro-2(3H)furanon hergestellt. Als Acetylierungsmittel verwendet man vorzugsweise Acetanhydrid oder Essigsäure selbst; grundsätzlich sind jedoch alle dem Fachmann für entsprechende Acetylierungen bekannten Acetylierungsmittel geeignet. Die Acetylierung wird in der Regel bei Temperaturen im Bereich von 40 °C bis 200 °C, vorzugsweise von 60 bis 140 °C über einen Zeitraum von 0,5 bis 10, vorzugsweise 0,8 bis 5 und insbesondere 1 bis 3 h durchgeführt.

Um eine vollständige Acetylierung sicherzustellen, wird das Acetylierungsmittel in der Regel in einem molaren Überschuß von 5. bis 50 %, vorzugsweise von 5 bis 20 %, bezogen auf das im Gemisch vorliegende 3-(2'-Hydroxyethyl)-dihydro-2(3H)furanon eingesetzt.

Nach einer ersten bevorzugten Ausführungsform des erfindungsgemaäßen Verfahrens wird zunächst die bei der Acetylierung als Koppelprodukt entstandene Essigsäure abdestilliert. Anschließend wird durch Zugabe eines geeigneten organischen Lösungsmittels und Wasser eine Phasentrennung vorgenommen und anschließend die organische Phase extrahiert. Als organische Lösungsmittel eignen sich insbesondere aromatische Kohlenwassertoffe und besonders alkylierte Benzolderivate wie Xylol oder Toluol. Nach Vereinigung der organischen Extrakte wird dann das Lösungsmittel durch eine vorgeschaltete Destillations- bzw. Rektifikationsstufe in mindestens einer Kolonne abgetrennt, bevor man die mehrstufige Destillation gemäß Anspruch 1 durchführt.

Nach einer weiteren bevorzugten Ausführungsform des erfindungsgemäßen Verfahrens wird das bei der Acetylierung entstandene Produkt mit starken Mineralsäuren behandelt, bevor es der Rektifikation bzw. Destillation unterworfen wird. Bevorzugte Mineralsäuren sind Salzsäure und besonders bevorzugt Schwefelsäure. Diese wird bevorzugt mindestens 80%ig und besonders in Form von konzentrierter Schwefelsäure eingesetzt.

Die Menge an eingesetzter Säure kann dabei in weiten Bereichen variiert werden; es hat sich in manchen Fällen als vorteilhaft herausgestellt, wenn das molare Verhältnis von starker Mineralsäure, bezogen auf die in der vorhergehenden Stufe eingesetzte Menge an Acetylierungsmittel im Bereich von 1: 20 bis 1:3, insbesondere von 1:15 bis 1:7 und insbesondere 1:8 bis 1:12 beträgt.

Die Temperatur bei der Behandlung mit der Mineralsäure liegt im allgemeinen im Bereich von 10 bis 80, vorzugsweise von 20 bis 60 und insbesondere von 30 bis 50 °C. Die Dauer der Behandlung liegt im allgemeinen im Bereich von 0,3 bis 10, insbesondere von 1 bis 5 h, kann aber grundsätzlich in weiten Grenzen variiert werden.

Nach der Behandlung mit starken Mineralsäuren lassen sich die Zersetzungsprodukte der unerwünschten Verunreinigungen, insbesondere des Dihydro-3-(2-methyl-1,3-dioxolan-2-yl)-2(3H)-furanon II einfach abtrennen. Besonders bevorzugt wird nach der Behandlung mit der Mineralsäure zunächst durch Zugabe einer Base die Mineralsäure neutralisiert und anschließend ggf. die bei der Acetylierung als Koppelprodukt entstandene Essigsäure wie in der ersten bevorzugten Ausführungsform beschrieben, abgetrennt. Anschließend erfolgt die mehrstufige Destillation bzw. Rektifikation gemäß Anspruch 1.

Die Reinheit von I nach Durchführung des erfindungsgemäßen Verfahrens liegt in der Regel bei mindestens 98, vorzugsweise bei mindestens 98,5 und besonders bevorzugt bei mindestens 99 Gew%.

Das nach dem erfindungsgemäßen Verfahren erhältliche I läßt sich in an sich bekannter Weise in hoher Ausbeute und mit guten Katalysatorstandzeiten zu Tetrahydropyran-4-carbonsäuremethylester umsetzen. Dieses ist ein wichtiges Vorprodukt bei der Herstellung von Pflanzenschutzmitteln.

### Beispiel 1 (Vergleich)

In einem 1 m³ Druckbehälter wurde eine Mischung aus 282,5 kg (2,44 kmol) Acetessigsäuremethylester, 340 l (271 kg) Methanol und 30,8 kg (0,17 kmol) einer 30%igen Natriummethylat-Lösung in Methanol vorgelegt und anschließend 214,4 kg (4,87 kmol) Ethylenoxid bei 60°C unter Rühren innerhalb von 8 Stunden zugepumpt. Danach wurde 24 h bei 60°C nachgerührt. Der Reaktionsaustrag wurde mehrfach mit Stickstoff gespült und anschließend in eine 1 m³ Rührapparatur mit aufgesetzter Kolonne überführt. Danach wurde der Katalysator durch Zugabe von 8,5 kg (83,3 mol) Schwefelsäure (96%) neutralisiert. Die niedrig siedenden Anteile, im wesentlichen Methanol, Methylacetat und Methylglykol, wurden bis zu einer Sumpftemperatur von 100°C bei 10 mbar (1013 Pa) abdestilliert. Zum Destillationsrückstand wurden bei 100°C innerhalb von einer Stunde unter Rühren 225,7 kg (2,21 kmol) Acetanhydrid gegeben und 2 h bei 100°C nachgerührt. Anschließend wurde das überschüssige Acetanhydrid sowie die als Koppelprodukt bei der Acetylierung entstandene Essigsäure abdestilliert und der Rohaustrag abgekühlt. Nach Zugabe von 350 kg Toluol und 150 kg Wasser wurden die Phasen getrennt und die wäßrige Phase zweimal mit je 200 kg Toluol extrahiert. Die organischen Extrakte wurden vereinigt und das Solvens im Vakuum abgezogen. Der Rückstand wurde im Vakuum bei 10 mbar diskontinuierlich rektifiziert (Sdp. 161 °C/10 mbar). Man erhielt 301,5 kg (72%) 3-(2'-Acetoxyethyl)-dihydro-2(3H)furanon (berechnet 100%).

### Zusammensetzung:

| | |
|---|---|
| 3-(2'-Acetoxyethyl)-dihydro-2(3H)furanon | 95,0%, |
| Dihydro-3-(2-methyl-1,3-dioxolan-2-yl)-2(3H)-furanon | 3,5% |
| 3-(2'-Hydroxyethyl)-dihydro-2(3H)furanon | 0,50% |
| Sonstige | 1,0% |

### Vergleichsbeispiel 2 (übliche mehrstufige Rektifikation, Abtrennung des Wertprodukts über Kopf)

In einem 1 m³- Druckbehälter wurde eine Mischung aus 282,5 kg (2,44 kmol) Acetessigsäuremethylester, 340 1 (271 kg) Methanol und 30,8 kg (0,17 kmol) einer 30 %igen Natriummethylat-Lösung in Methanol vorlegt und anschließend 214,4 kg (4,87 kmol) Ethylenoxid bei 60°C unter Rühren innerhalb von 8 Stunden zugepumpt. Danach wurde 24 h bei 60°C nachgerührt. Der Reaktionsaustrag wurde mehrfach mit Stickstoff gespült und anschließend in eine 1 m³-Rührapparatur mit aufgesetzter Kolonne überführt. Danach wurde der Katalysator durch Zugabe von 8,5 kg (83,3 mol) Schwefelsäure (96%) neutralisiert. Die niedrig siedende Anteile, im wesentlichen Methanol, Methylacetat und Methylglykol, wurden bis zu einer Sumpftemperatur von 100°C bei 10 mbar abdestilliert. Zum Destillationsrückstand wurden bei 100°C innerhalb von einer Stunde unter Rühren 225,7 kg (2,21 kmol) Acetanhydrid gegeben und 2 h bei 100°C nachgerührt. Anschließend wurde das überschüssige Acetanhydrid sowie die als Koppelprodukt bei der Acetylierung entstandene Essigsäure abdestilliert und der Rohaustrag abgekühlt. Nach Zugabe von 350 kg Toluol und 150 kg Wasser wurden die Phasen getrennt und die wäßrige Phase zweimal mit je 200 kg Toluol extrahiert. Die organischen Extrakte wurden vereinigt und wie folgt weiter aufgearbeitet: In einer ersten kontinuierlich betriebenen Kolonne (Durchmesser: 43 mm, gepackt mit 1m Sulzerpackungen BX) wurde das Toluol in einer Reinheit von 99,9 Gew.% bei 300 mbar Kopfvakuum abdestilliert und das toluolfreie Wertprodukt im Sumpf abgezogen. Von dort aus wurde das Wertprodukt in eine zweite kontinuierlich betriebene Kolonne (Durchmesser: 43 mm, gepackt mit 2,4 m Sulzerpackungen CY) überführt, in der Leicht- und Zwischensieder über Kopf (Kopfdruck: 10 mbar) abgetrennt wurden. Das Sumpfprodukt wurde in einer dritten kontinuierlich betriebenen Kolonne (Durchmesser: 43 mm, gepackt mit 2,4 m Sulzerpackungen CY) von Schwersiedern befreit (Kopfdruck: 10 mbar) und als Kopfprodukt (Sdp. 161°C/10 mbar) gewonnen.

Man erhielt 303,7 kg (72%) 3-(2'-Acetoxyethyl)-dihydro-2(3H)furanon (berechnet 100%).

### Zusammensetzung:

| | |
|---|---|
| 3-(2'-Acetoxyethyl)-dihydro-2(3H)furanon | 98,9% |
| Dihydro-3-(2-methyl-1,3-dioxolan-2-yl)-2(3H)-furanon | 0,1% |
| 3-(2'-Hydroxyethyl)-dihydro-2(3H)furanon | 0,3% |
| Sonstige | 0,6% |

### Beispiel 3 (erfindungsgemäß (dreifache kont. Rektifikation, Abtrennung des Wertprodukts aus dem Sumpf))

In einem 1 m³- Druckbehälter wurde eine Mischung aus 282,5 kg (2,44 kmol) Acetessigsäuremethylester, 340 l (271 kg) Methanol und 30,8 kg (0,17 kmol) einer 30 %igen Natriummethylat-Lösung in Methanol vorlegt und anschließend 214,4 kg (4,87 kmol) Ethylenoxid bei 60°C unter Rühren innerhalb von 8 Stunden zugepumpt. Danach wurde 24 h bei 60°C nachgerührt. Der Reaktionsaustrag wurde mehrfach mit Stickstoff gespült und anschließend in eine 1 m³-Rührapparatur mit aufgesetzter Kolonne überführt. Danach wurde der Katalysator durch Zugabe von 8,5 kg (83,3 mol) Schwefelsäure (96%) neutralisiert. Die niedrig siedende Anteile, im wesentlichen Methanol, Methylacetat und Methylglykol, wurden bis zu einer Sumpftemperatur von 100°C bei 10 mbar abdestilliert. Zum Destillationsrückstand wurden bei 100°C innerhalb von einer Stunde unter Rühren 225,7 kg (2,21 kmol) Acetanhydrid gegeben und 2 h bei 100°C nachgerührt. Anschließend wurde das überschüssige Acetanhydrid sowie die als Koppelprodukt bei der Acetylierung entstandene Essigsäure abdestilliert und der Rohaustrag abgekühlt. Nach Zugabe von 350 kg Toluol und 150 kg Wasser wurden die Phasen getrennt und die wäßrige Phase zweimal mit je 200 kg Toluol extrahiert. Die organischen Extrakte wurden vereinigt und wie folgt weiter aufgearbeitet: In einer ersten kontinuierlichen Kolonne (Durchmesser: 43 mm, gepackt mit 1 m Sulzerpackungen BX) wurde das Toluol bei 300 mbar Kopfvakuum in einer Reinheit von 99,9% abdestilliert und das toluolfreie Wertprodukt im Sumpf abgezogen. Von dort aus wurde das Wertprodukt in eine zweite kontinuierliche Kolonne (Durchmesser: 43 mm, gepackt mit 2,4 m Sulzerpackungen CY) überführt, in der Hochsieder abgetrennt wurden (Kopfdruck: 10mbar). Das Kopfprodukt wurde nun in einer dritten kontinuierlichen Kolonne (Durchmesser: 43 mm, gepackt mit 2,4 m Sulzerpakkungen CY) von Leicht- und Zwischensiedern befreit (Kopfdruck: 10mbar) und im Sumpf der Kolonne gewonnen (Sdp. 161°C/10 mbar). Man erhielt 304,5 kg (73%) 3-(2'-Acetoxyethyl)-dihydro-2(3H)furanon (berechnet 100%).

### Zusammensetzung:

| | |
|---|---|
| 3-(2'-Acetoxyethyl)-dihydro-2(3H)furanon | 99,5% |
| Dihydro-3-(2-methyl-1,3-dioxolan-2-yl)-2(3H)-furanon | <0,1% |
| 3-(2'-Hydroxyethyl)-dihydro-2(3H)furanon | 0,3% |
| Sonstige | 0,1% |

Um die Auswirkungen des Reinheitsgrades von I auf die nachfolgende Umsetzung zu untersuchen, wurden die nach den Beispielen 1 bis 3 erhaltenen Produkte zu Tetrahydropyran-4-carbonsäuremethylester gemäß folgender allgemeiner Gleichung umgesetzt:

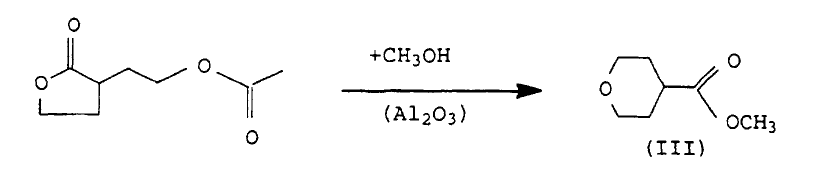

Pro Stunde wurde eine Lösung bestehend aus 172 g 3-(2'-Acetoxyethyl)-dihydro-2(3H)furanon und 192 g Methanol verdampft und in einem Rohrreaktor bei 250°C über 2000 g eines γ-Aluminiumoxid-Katalysators (Pural SB™, zu 2 mm Strängen verformt, bei 120°C/16 h getrocknet und 520°C/3 h calciniert) geleitet. Der gasförmige Reaktionsaustrag wurde kondensiert und der entstandene Tetrahydropyran-4-carbonsäureester durch diskontinuierliche Destillation gereinigt (Sdp. 117°C/30 mbar)

Die Ergebnisse der Umsetzungen von 3-(2'-Acetoxyethyl)-dihydro-2(3H)furanon I mit verschiedenen Anteilen Dihydro-3-(2-methyl-1,3-dioxolan-2-yl)-2(3H)-furanon II sind in Tabelle 1 zusammengefaßt (der Umsatz betrug in allen Fällen 100 %) :

**Tabelle 1:**

| Anteil II in % | Reinheit von I, in % | Ausbeute an III, zu Beginn | Ausbeute an III, nach 60 h | Ausbeute an III, nach 1200h | Ausbeute an III, nach 1440h |
|---|---|---|---|---|---|
| 3,5 (Bsp.1) | 95,0 | 55% | 39% | - | - |
| 0,1 (Bsp.2) | 98,9 | 68% | 68% | 68% | 62% |
| <0,1 (Bsp.3) | 99,5 | 68% | 68% | 68% | 68% |

Die Ergebnisse der vorstehenden Tabelle zeigen den starken Einfluß des Gehalts an Verunreinigungen, insbesondere II, auf die weitere Verarbeitung von I.

## Patentansprüche

1. Verfahren zur Abtrennung von Verunreinigungen aus 3-(2'-Acetoxyethyl)-dihydro-2(3H)furanon (I), wobei das die unerwünschten Verunreinigungen enthaltende 3-(2'-Acetoxyethyl)-dihydro-2(3H)furanon zunächst in an sich bekannter Weise durch Acetylierung von 3-(2'-Hydroxyethyl)-dihydro-2(3H)furanon hergestellt wird und anschließend das so erhaltene Produkt einer Destillation oder Rektifikation unterworfen wird, **dadurch gekennzeichnet, daß** die Destillation oder Rektifikation mehrstufig durchgeführt wird, wobei schwersiedende Verunreinigungen in einer ersten Stufe abgetrennt werden, anschließend das über Kopf abgezogene Produkt mindestens einer weiteren Stufe unterworfen wird, in der Leicht- und Zwischensieder über Kopf abgezogen und das gewünschte reine I als Sumpfprodukt erhalten wird.

2. Verfahren nach Anspruch 1, **dadurch gekennzeichnet, daß** man das nach der Acetylierung erhaltene Produkt einer vorgeschalteten Destillation oder Rektifikation zur Abtrennung der als Koppelprodukt bei der Acetylierung anfallenden Essigsäure unterwirft und dann durch Zugabe eines organischen Lösungsmittels extrahiert.

3. Verfahren nach einem der Ansprüche 1 oder 2, **dadurch gekennzeichnet, daß** man das nach der Acetylierung erhaltene Produkt vor der ersten Destillation oder Rektifikation mit einer starken Mineralsäure behandelt.

4. Verfahren nach Anspruch 3, **dadurch gekennzeichnet, daß** man als starke Mineralsäure Schwefelsäure verwendet.

5. Verfahren nach einem der Ansprüche 3 oder 4, **dadurch gekennzeichnet, daß** man die Behandlung mit Schwefelsäure bei Temperaturen von 20 bis 70 °C durchführt.

## Claims

1. A process for removing impurities from 3-(2'-acetoxyethyl)-dihydro-2(3H)-furanone (I), where the 3-(2'-acetoxyethyl)-dihydro-2(3H)-furanone containing the undesirable impurities is initially prepared in a manner known per se by acetylating 3-(2'-hydroxyethyl)-dihydro-2-(3H)-furanone and the resulting product is subsequently subjected to distillation or rectification, wherein the distillation or rectification is carried out in a plurality of steps where high-boiling impurities are removed in a first step and the product drawn off via the top is then subjected to at least one further step in which low boilers and intermediate boilers are drawn off via the top and the desired pure I is obtained as bottom product.

2. A process as claimed in claim 1, wherein the product obtained after the acetylation is subjected to an upstream distillation or rectification to remove the acetic acid which is obtained as coproduct during the acetylation and is then extracted by adding an organic solvent.

3. A process as claimed in claim 1 or 2, wherein the product obtained after the acetylation is treated with a strong mineral acid prior to the first distillation or rectification.

4. A process as claimed in claim 3, wherein the strong mineral acid used is sulfuric acid.

5. A process as claimed in either of claims 3 or 4, wherein the treatment with sulfuric acid is carried out at temperatures of from 20 to 70°C.

## Revendications

1. Procédé pour séparer les impuretés de la 3-(2'-acétoxyéthyl)- dihydro-2(3H)-furannone (I) dans lequel on prépare d'abord de manière connue en soi la 3-(2'-acétoxyéthyl)-dihydro-2(3H)furannone contenant les impuretés gênantes par acétylation de la 3-(2'-hydroxyémyl)-dihydro-2(3H)mrannone et on soumet ensuite le produit ainsi obtenu à une distillation ou rectification, **caractérisé par le fait que** la distillation ou rectification est réalisée en plusieurs stades opératoires : dans un premier stade opératoire, on sépare les impuretés à haut point d'ébullition, on soumet ensuite le produit évacué en tête à au moins un autre stade opératoire dans lequel on évacue en tête les produits à bas point d'ébullition et les produits à point d'ébullition intermédiaire et on obtient le composé I pur recherché en pied de colonne.

2. Procédé selon la revendication 1, **caractérisé par le fait que** l'on soumet le produit obtenu après acétylation à une distillation ou rectification préalable pour séparation de l'acide acétique formé en produit d'accompagnement à l'acétylation et on extrait ensuite par addition d'un solvant organique.

3. Procédé selon l'une des revendications 1 ou 2, **caractérisé par le fait que** l'on traite le produit obtenu après acétylation à traitement par un acide minéral fort avant la première distillation ou rectification.

4. Procédé selon la revendication 3, **caractérisé par le fait que** l'acide minéral fort utilisé est l'acide sulfurique.

5. Procédé selon l'une des revendications 3 et 4, **caractérisé par le fait que** le traitement par l'acide sulfurique est réalisé à des températures de 20 à 70°C.
